# EUROPÄISCHE PATENTSCHRIFT

(11) **EP 3 250 247 B1**
(45) Veröffentlichungstag und Bekanntmachung des Hinweises auf die Patenterteilung: **29.08.2018**
(21) Anmeldenummer: 16702899.2
(22) Anmeldetag: 27.01.2016
(51) Int. Cl.: A61L 27/04, A61L 27/58, A61L 31/02, A61L 31/14, C22C 18/00

(54) **BIODEGRADIERBARE LEGIERUNG SOWIE DEREN HERSTELLUNG UND VERWENDUNG, INSBESONDERE ZUR HERSTELLUNG VON STENTS UND ANDEREN IMPLANTATEN**
BIODEGRADABLE ALLOY, PRODUCTION AND USE THEREOF, IN PARTICULAR FOR PRODUCING STENTS AND OTHER IMPLANTS
ALLIAGE BIODÉGRADABLE, FABRICATION ET UTILISATION DUDIT ALLIAGE BIODÉGRADABLE, EN PARTICULIER POUR LA FABRICATION D'ENDOPROTHÈSES ET AUTRES IMPLANTS

(30) Priorität: 28.01.2015 DE 102015101264
(43) Veröffentlichungstag der Anmeldung: 06.12.2017
(73) Patentinhaber: Limedion GmbH, 68163 Mannheim (DE)
(72) Erfinder: KOVACS, Adalbert, 68526 Ladenburg (DE); SCHIESTEL, Stefanie, 64653 Lorsch (DE)
(74) Vertreter: Beyer, Carsten
(86) Internationale Anmeldenummer: PCT/EP2016/051720
(87) Internationale Veröffentlichungsnummer: WO 2016/120338

(56) Entgegenhaltungen:
- EP-A1- 1 840 235
- EP-A2- 0 966 979
- DE-A1- 19 729 545
- US-A1- 2013 284 392
- DATABASE WPI Week 201522 Thomson Scientific, London, GB; AN 2015-10910Q XP002756113, & CN 104 212 998 A (UNIV PEKING) 17. Dezember 2014 (2014-12-17)
- DATABASE WPI Week 201522 Thomson Scientific, London, GB; AN 2015-08891W XP002756120, & CN 104 195 369 A (UNIV PEKING) 10. Dezember 2014 (2014-12-10) -& DATABASE EPODOC [Online] EUROPEAN PATENT OFFICE, THE HAGUE, NL; XP002756121, Database accession no. CN-201410415525-A & CN 104 195 369 A (UNIV BEIJING) 10. Dezember 2014 (2014-12-10)
- DATABASE WPI Week 200429 Thomson Scientific, London, GB; AN 2004-312205 XP002756128, & KR 2003 0094919 A (KOREA INST MACHINERY & MATERIALS) 18. Dezember 2003 (2003-12-18) -& DATABASE EPODOC [Online] EUROPEAN PATENT OFFICE, THE HAGUE, NL; XP002756129, Database accession no. KR-20020032269-A & KR 2003 0094919 A (KOREA MACHINERY & METAL INST [KR]) 18. Dezember 2003 (2003-12-18)
- PATRICK K. BOWEN ET AL: "Zinc Exhibits Ideal Physiological Corrosion Behavior for Bioabsorbable Stents", ADVANCED MATERIALS, Bd. 25, Nr. 18, 14. Mai 2013 (2013-05-14), Seiten 2577-2582, XP055224983, DE ISSN: 0935-9648, DOI: 10.1002/adma.201300226

## Beschreibung

Die vorliegende Erfindung betrifft eine biodegradierbare Legierung sowie deren Herstellung und Verwendung, insbesondere zur Herstellung von Stents und anderen Implantaten. Des Weiteren betrifft die Erfindung Implantate, vorzugsweise Stents oder Knochenimplantate, welche die erfindungsgemäße biodegradierbare Legierung aufweisen.

Metallische Stents aus CoCr, Edelstahl oder Nitinol (eine Nickel-Titan-Legierung mit Formgedächtnis) werden seit über drei Jahrzehnten zur Behandlung von verengten Blutgefäßen (Stenosen) eingesetzt. Aufgrund ihrer hohen Korrosionsbeständigkeit verbleiben diese Implantate in der Regel ein Leben lang im Körper. Die stützende Wirkung der Endoprothesen ist aber aufgrund des Heilungsprozesses nach einer bestimmten Zeit (1-2 Jahre) nicht mehr notwendig, wodurch sich der Einsatz von biodegradierbaren (bioresorbierbaren) Implantaten anbietet, welche die Nachteile eines lebenslangen Implantats dadurch umgehen, dass sie sich nach der Erfüllung ihrer Funktion im Körper auflösen.

Stand der Technik bei bioresorbierbaren Stents sind u.a. Stents aus polymeren Werkstoffen, insbesondere aus Poly-Milchsäure (PLA), und insbesondere aus Poly-L-Milchsäure (PLLA). Derartige Stents sind bspw. aus WO 2009/155206 A2 und US 2013/0338762 A1 bekannt. Polymere Werkstoffe und insbesondere Polymilchsäure können jedoch u.a. Nachteile bei der mechanischen Stabilität des Stents zeigen.

Ebenfalls bekannt sind bioresorbierbare Stents aus Legierungen auf Magnesiumbasis, so bspw. aus EP 1 886 651 A1. Ebenfalls bekannt sind Stents, welche aus der Magnesiumlegierung WE43 gefertigt sind. Diese Legierung ist u.a. in WO 2010/038016 A1 beschrieben. Legierungen auf Mg-Basis können sich jedoch unter Umständen aufgrund einer zu raschen Auflösungsgeschwindigkeit (bspw. bereits nach 6 Monaten) des Stents, unbefriedigender mechanischer Eigenschaften und auch einer unerwünschten Freisetzung von Wasserstoff während der Auflösung als nachteilig erweisen.

Eine mögliche kritische Wasserstoffentwicklung und/oder Wasserstoffakkumulation bei in einem lebenden Körper eingebrachten Implantaten wird dabei in EP 2 014 317 A2 anhand von Implantaten mit einer magnesiumhaltigen Schicht beschrieben.

Die Unzulänglichkeiten bekannter Stents und hierfür verwendeter Werkstoffe führen dazu, dass diese verbreitet mit Medikamenten beschichtet werden. Ein Nachteil solcher Produkte kann darin bestehen, dass sie antiproliferative Medikamente, wie Everolimus oder Paclitaxel freisetzen, die eine zwölfmonatige Begleittherapie mit ASS und Clopidogrel zwingend erforderlich machen. Genannte Medikamente der Stentbeschichtung sind cytotoxisch und werden auch in der Krebstherapie oder bei der Verhinderung einer Transplantatabstoßung eingesetzt.

CN-A-104212998 (XP002756113) und CN-A-104195369 (XP002756120/XP002756121) beschreiben für medizinische Implantate geeignete, ternäre biodegradierbare Magnesium bzw. Calcium enthaltende Zink-Legierungen die als Mikroelement unter anderem Silber enthalten.

Der vorliegenden Erfindung liegt die Aufgabe zugrunde, zumindest eines der obigen Probleme zu umgehen.

Des Weiteren liegt der Erfindung die Aufgabe zugrunde, eine verbesserte biodegradierbare Legierung und ein Verfahren zu deren Herstellung anzugeben.

Ferner liegt der Erfindung die Aufgabe zugrunde, eine biodegradierbare Legierung mit verbesserten Eigenschaften bei der Verwendung in Stents oder sonstigen Implantaten anzugeben.

Schließlich liegt der Erfindung die Aufgabe zugrunde, einen Beitrag zu einer verbesserten Versorgung mit Stents und/oder anderen Implantaten zu leisten.

Die Erfindung wird durch die Ansprüche definiert. Einen Beitrag zur Lösung mindestens einer der vorstehend genannten Aufgaben leistet eine biodegradierbare Legierung mit den Merkmalen des Anspruchs 1.

Vorteilhafte Ausgestaltungen und Weiterbildungen der erfindungsgemäßen biodegradierbaren Legierung ergeben sich aus den hierzu nachgeordneten Ansprüchen.

Einen Beitrag zur Lösung mindestens einer der vorstehend genannten Aufgaben leisten ferner eine Verwendung der erfindungsgemäßen Legierung nach den Merkmalen des Anspruchs 6 sowie ein Implantat, welches eine erfindungsgemäße Legierung aufweist, nach Anspruch 7.

Einen Beitrag zur Lösung mindestens einer der vorstehend genannten Aufgaben leistet schließlich ein Verfahren zur Herstellung einer biodegradierbaren Legierung nach Anspruch 9.

Vorteilhafte Ausgestaltungen und Weiterbildungen der vorgenannten weiteren Aspekte der Erfindung ergeben sich aus den jeweils hierzu nachgeordneten Ansprüchen.

Im Rahmen der vorliegenden Erfindung wird zunächst eine biodegradierbare Legierung mit den Merkmalen des Anspruchs 1 vorgeschlagen.

Danach wird eine biodegradierbare Legierung mit einem hohen Zinkanteil vorgeschlagen, wobei die erfindungsgemäße Legierung aus Zink und Silber besteht und (in Massen-%) 90,0-99,95% Zink und 0,05-10% Silber aufweist. Die erfindungsgemäße Legierung eignet sich insbesondere als Werkstoff oder als Beschichtungsmaterial für Implantate, bevorzugt für Stents.

Zunächst ist Zink als biokompatibel anzusehen, und muss dem menschlichen Körper als Spurenelement zugeführt werden. Es wirkt anti-proliferativ und reduziert somit das überschießende Muskelzellwachstum, das zur Intimahyperplasie und dadurch zu einer erneuten Verengung des Blutgefäßes (Restenose) führt.

Neben der antiproliferativen Wirkung zeigt Zink auch antithrombotische und antibakterielle Eigenschaften, weshalb es im allgemeinen Fall als nicht notwendig angesehen wird, zinkbasierte Implantate/Stents mit einer Medikamentenbeschichtung zu versehen.

Die mechanischen Eigenschaften der erfindungsgemäßen Zinklegierung werden als vorteilhaft im Vergleich mit bekannten Stentmaterialien angesehen, so bspw. im Vergleich zu PLLA oder auch zu bekannten Magnesium-Legierungen.

Auf der anderen Seite zeigen Implantate (Stents) bestehend aus der erfindungsgemäßen Zinklegierung auch bessere mechanische Eigenschaften als Implantate aus reinem Zink.

Des Weiteren zeigt die erfindungsgemäße biodegradierbare Legierung keine nennenswerte Wasserstoffentwicklung bei der Auflösung im Körper, wie sie bspw. in EP 2 014 317 A2 beschrieben wird.

Die Auflösungskinetik der erfindungsgemäßen Legierung in vivo liegt deutlich unter derjenigen von bspw. Mg-Legierungen und kann üblicherweise die allgemein angestrebten 1 - 2 Jahre bis zur Auflösung des Implantats bereitstellen.

Es hat sich ferner gezeigt, dass erfindungsgemäße Legierungen aufgrund ihrer im Vergleich zu Mg (1,74 g/cm³) und PLLA (1,2-1,4 g/cm³) hohen Dichte (7,1-7,2 g/cm³) im Röntgen sehr gut sichtbar sind. Die Röntgensichtbarkeit ist für den behandelnden Arzt bei der Platzierung des Stents an der verengten Gefäßstelle enorm wichtig. Bei Mg und PLLA muss diese Sichtbarkeit durch Anbringen eines röntgendichten Materials am Stent (Au, Pt oder Ta) z.B. als dünnes, aufgepresstes Plättchen erzielt werden, was beim Auflösen des Stents eventuell zu Problemen führen kann, da das Plättchen dann in den Blutkreislauf gelangen kann.

Mit den Merkmalen des Anspruchs 6 wird die Verwendung einer erfindungsgemäßen biodegradierbaren Legierung zur Herstellung oder Beschichtung eines Implantats, insbesondere eines Stents oder eines Knochenimplantats, vorgeschlagen.

Besonders bevorzugt ist dabei die Verwendung einer erfindungsgemäßen Legierung zur Herstellung eines Stents. Vorzugsweise wird der Stent dabei vollständig aus der erfindungsgemäßen Legierung hergestellt.

Mit den Merkmalen des Anspruchs 7 wird ferner ein Implantat vorgeschlagen, welches aus einer erfindungsgemäßen biodegradierbaren Legierung hergestellt oder mit einer solchen Legierung beschichtet ist.

Bevorzugt weist das erfindungsgemäß vorgeschlagene Implantat einen Stent auf, der teilweise oder vollständig, und vorzugsweise vollständig, aus der erfindungsgemäßen biodegradierbaren Legierung hergestellt sein kann. Alternativ, jedoch ebenfalls bevorzugt, kann das erfindungsgemäße Implantat ein Knochenimplantat aufweisen.

Mit den Merkmalen des Anspruchs 9 wird schließlich ein Verfahren zur Herstellung der erfindungsgemäßen biodegradierbaren Legierung vorgeschlagen. Dieses umfasst die Schritte:
(a) Schmelzen der Legierungsbestandteile unter Schutzgas
(b) Vibrieren der Gussform während des Erstarrens der Schmelze
(c) Erwärmen der Legierung und Halten der Temperatur für definierte Zeiten f
(d) Abschrecken der Legierung
(e) Auslagern der Legierung bei definierten Temperaturen f
(f) Mechanisches Verformen

Mit den Schritten (a) und (b) wird dabei eine Mischkristallverfestigung sowie eine Korngrenzenverfestigung erreicht.

Im Folgenden werden die erfindungsgemäßen Aspekte weiter erörtert, wozu teilweise auf nicht limitierende Ausführungsbeispiele sowie vorteilhafte Ausgestaltungen und Weiterbildungen der Erfindung Bezug genommen wird. Die nachstehend erörterten vorteilhaften Ausgestaltungen und Weiterbildungen lassen sich dabei alleine oder wahlweise auch in jeder beliebigen Kombination realisieren.

Eine Zusammenstellung von ergänzenden bzw. zusätzlichen, nicht limitierenden, konkreten bevorzugten Ausführungsbeispielen der erfindungsgemäßen Legierung findet sich überdies im folgenden Abschnitt. Auf diese Zusammenstellung wird ausdrücklich verwiesen.

Im Rahmen des erfindungsgemäßen Verfahrens wird hinsichtlich einer weiteren Erhöhung der mechanischen Stabilität der Legierung eine Aushärtung der Legierung im Rahmen der zusätzlichen Schritte (c) bis (e) vorgeschlagen:
(c) Erwärmen der Legierung und Halten der Temperatur für definierte Zeiten
(d) Abschrecken der Legierung
(e) Auslagern der Legierung bei definierten Temperaturen

Hieran schließt sich ein weiterer Schritt mit einer Umformung, vorzugsweise einer Warmumformung, an:
(f) Mechanisches Verformen

Eine besonders bevorzugte Ausführungsform des erfindungsgemäßen Verfahrens gemäß der vorteilhaften Ausgestaltung nach den vorstehenden Schritten (a) bis (f) weist dabei die nachfolgend aufgeführten konkretisierten Schritte (a1) bis (f1), insbesondere in der genannten Reihenfolge, auf, wobei jedoch auch die Realisierung einer oder einer Auswahl der Schritte (a1) bis (f1) innerhalb eines Herstellungsverfahrens nach Schritt (a) bis (f) als vorteilhaft angesehen wird:
(a1) Schmelzen der Legierungsbestandteile unter Einsatz eines Schutzgases, vorzugsweise Argon, und vorzugsweise unter Verwendung eines leichten Überdrucks, insbesondere von 1,1-1,3 bar
(b1) Vibrieren der Gussform während des Erstarrens der Schmelze; dadurch kann eine homogene Verteilung der Legierungsbestandteile sowie eine Korngrößenreduzierung (Kornfeinung) erreicht werden
(c1) Erwärmen der Legierung in einer Argonatmosphäre bei 100°C-300°C und einer Haltezeit von 0,1 bis 10 Stunden
(d1) Abschrecken der Legierung in einem flüssigen Kühlmedium, z.B. Wasser, vorzugsweise bei Raumtemperatur
(e1) Auslagern der Legierung in einer Schutzgasatmosphäre, vorzugsweise Argon, bevorzugt bei 50°C bis 150°C, insbesondere für 0,1 bis 1 Stunde
(f1) Warmumformung, bevorzugt bei 300°C bis 400°C, vorzugsweise durch Walzen, Strangpressen oder Rundkneten

In einer vorteilhaften Weiterbildung des Verfahrens ist zwischen Schritt (a) und Schritt (b) bzw. zwischen Schritt (a1) und Schritt (b1) die Zugabe eines Zinkreinigungsmittels als Additiv vorgesehen. Ein solches Additiv kann zur Reinigung der Zinkschmelze beitragen. Es wirkt durch Lösen der in der Zinkschmelze enthaltenen Metalloxide durch Reduktion. Des Weiteren kann die Benetzungsfähigkeit des geschmolzenen Zinks und die Korrosionsbeständigkeit erhöht werden. Ein hierfür geeignetes Zinkreinigungsmittel kann bspw. von der Fa. Dipl.-Ing. Herwig GmbH, 58093 Hagen, DE, unter der Produktbezeichnung Hega Power® bezogen werden.

Nachfolgend werden nunmehr weitere Aspekte der vorgeschlagenen Legierung erörtert. Erfindungsgemäß vorgesehen ist zunächst eine biodegradierbare Legierung, wobei die Legierung besteht aus (in Massen-% / Ma.-%): Zink (Zn): 90,0 - 99,95%; Silber: 0,05 - 10,0%.

In einer ersten vorteilhaften Weiterbildung besteht die biodegradierbare Legierung aus: Zink (Zn): 90,0 - 99,5%; Silber: 0,5 - 10,0%.

Weiter bevorzugt besteht die biodegradierbare Legierung aus: Zink (Zn): 90,0 - 99,0%; Silber: 1,0 - 10,0%; und besteht insbesondere aus: Zink (Zn): 91,0 - 99,0%; Silber: 1,0 - 9,0%.

In einer nächsten vorteilhaften Weiterbildung besteht die biodegradierbare Legierung aus Zink und Silber (Ag), insbesondere mit einem Silbergehalt von 0,1 - 6,0 Ma.-%, und insbesondere mit einem Silbergehalt von 0,9 - 4,0 Ma.-%. Besonders bevorzugt ist dabei ein Silbergehalt von 1,0 Ma.-%.

Eine bevorzugte Ausführungsform der erfindungsgemäßen Legierung besteht aus Zink und Silber, mit einem Silbergehalt von 0,1 - 6,0 Ma.-%, bevorzugt 0,9 - 4,0 Ma.-%, besonders bevorzugt 1,0 Ma.-%.

Als besonders vorteilhaft erweist sich eine Ausgestaltung der erfindungsgemäßen biodegradierbaren Legierung, insbesondere einer Legierung nach einer oder mehreren der vorstehenden Ausführungsformen, welche eine oder mehrere der folgenden Eigenschaften (a) bis (g) aufweist:
(a) Brinellhärte HBW 2,5/62,5 > 50
(b) Dehngrenze (Rp 0,2) > 100 MPa
(c) Zugfestigkeit Rm > 200 MPa
(d) Bruchdehnung > 5%
(e) Auflösezeit im Körper beträgt 1 - 2 Jahre
(f) keine Gefahr einer Emboliebildung im Körper durch Wasserstoffentwicklung
(g) keine Gewebeschädigung im Körper durch Wasserstoffakkumulation

Zur Definition einer kritischen Wasserstoffentwicklung und/oder Wasserstoffakkumulation bei in einem lebenden Körper eingebrachten Implantaten wird dabei auf EP 2 014 317 A2 verwiesen.

Ferner wird vorgeschlagen die Verwendung einer erfindungsgemäßen biodegradierbaren Legierung, insbesondere einer Legierung nach einer oder mehreren der vorstehenden Ausführungsformen, zur Herstellung oder Beschichtung eines Implantats, insbesondere eines Stents oder eines Knochenimplantats.

Ein erfindungsgemäßes Implantat ist aus einer erfindungsgemäßen biodegradierbaren Legierung, insbesondere einer Legierung nach einer oder mehreren der vorstehenden Ausführungsformen, hergestellt oder mit einer solchen Legierung beschichtet.

Eine vorteilhafte Weiterbildung des erfindungsgemäßen Implantats weist einen Stent oder ein Knochenimplantat auf.

### Beispiele

Ausführungsbeispiele der Erfindung, welche die Erfindung jedoch nicht auf die dargestellten Beispiele einschränken, werden im Rahmen der folgenden tabellarischen Aufstellung von beispielhaften Zusammensetzungen der erfindungsgemäßen Legierung in der Zusammenschau mit nicht erfindungsgemäßen Vergleichsbeispielen nach dem Stand der Technik gezeigt. Die beispielhaften Zusammensetzungen gemäß unten stehender **Tabelle 1** geben gleichzeitig - zusätzlich oder ergänzend zum Gegenstand der nachgeordneten Ansprüche - besonders bevorzugte Ausführungsformen der erfindungsgemäßen Legierung wieder. Die Beispiele 2-6 sind Vergleichsbeispiele und nicht erfindungsgemäß.

| Beispiel | Zusammensetzung | Härtewert HBW 2,5/62,5 | Dehngrenze Rp0,2 [MPa] | Zugfestigkeit Rm [MPa] | Bruchdehnung [%] |
|---|---|---|---|---|---|
| V1 | Zink | 33 | 47 | 80 | 8 |
| V2 | WE43 | 80 | 162 | 220 | 2 |
| V3 | PLLA | - | 60 | 28-70 | 2-6 |
| 1 | ZnAg1 | 60 | 138 | 196 | 25 |
| 2 | ZnTi1 | 56 | 212 | 259 | 10 |
| 3 | ZnAl4Cu3 | 130 | 361 | 397 | 6 |
| 4 | ZnAl8Cu1 | 110 | 319 | 387 | 8 |
| 5 | ZnAg0,9Mg0,9 | 70 | 230 | 265 | 9 |
| 6 | ZnMg1,0Al1,0 | 85 | 234 | 260 | 10 |

Zur Kennzeichnung aller weiteren Legierungen, insbesondere der erfindungsgemäßen Legierungen, wird hier und innerhalb des gesamten Dokuments stets eine Legierungsformel gemäß DIN 1310 verwendet. Dabei wird zuerst das Basismetall genannt, dahinter folgen der oder die weiteren Bestandteile als Kürzel für das Metall mit angehängter Zahl, die den jeweiligen Prozentanteil in Massen-% (Ma.-%) an der Legierung wiedergibt.

Die in Tabelle 1 aufgeführten Werte der Brinellhärte sind gemäß nach dem Prüfverfahren HBW 2,5/62,5 (Angabe gemäß EN ISO 6506-1) ermittelt worden Die Zugversuche zur Bestimmung der mechanischen Werte Dehngrenze, Zugfestigkeit und Bruchdehnung sind nach EN ISO 6892-1 durchgeführt worden.

Die in **Tabelle 1** dargestellten Messwerte für die mechanischen Eigenschaften der erfindungsgemäßen Legierung gemäß Beispiel 1 sowie den nicht erfindungsgemäßen Legierungen gemäß den Beispielen 2 bis 6 zeigen im Vergleich mit den Werten für die nicht erfindungsgemäßen Vergleichsbeispiele V1 bis V3 im Einzelnen folgendes Bild:
In Bezug auf die Werte der Brinellhärte liegen die Ergebnisse des erfindungsgemäßen Beispiels 1 sowie sämtlicher nicht erfindungsgemäßer Beispiele 2 bis 6 deutlich oberhalb des Wertes, welche für reines Zink gemessen wird. Die Legierungen ZnAg1 sowie ZnTi1 (Beispiele 1 und 2) zeigen dabei Härtewerte, welche etwas unterhalb des Wertes für die Mg-Legierung WE43 (Vergleichsbeispiel V2) angesiedelt ist. Die Härtewerte für ZnAg0,9Mg0,9 (Beispiel 5) sowie ZnMg1,0Al1,0 (Beispiel 6) liegen im Bereich des Wertes für WE43. Die nicht erfindungsgemäßen Beispiellegierungen ZnAl4Cu3 sowie ZnAl8Cu1 (Beispiele 3 und 4) zeigen hingegen Härtewerte, welche deutlich oberhalb des Wertes für WE 43 angesiedelt sind.

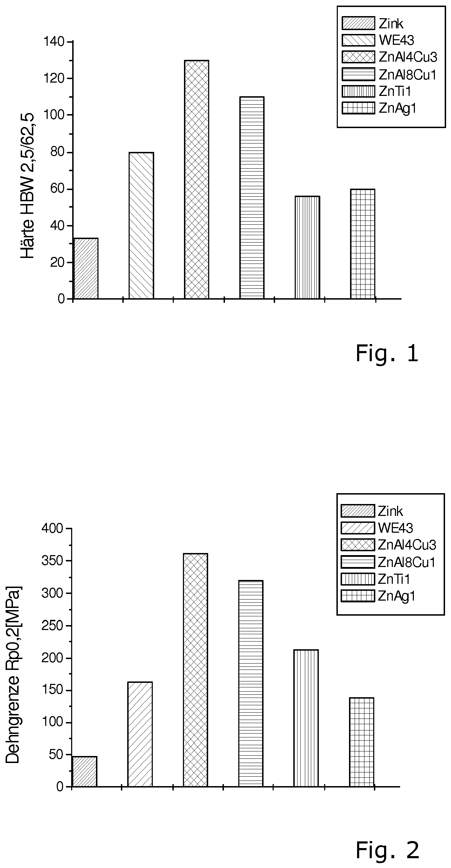

Die in Tab. 1 in Bezug auf die Härtewerte gezeigten Ergebnisse sind überdies auszugsweise in **Figur 1** grafisch aufgetragen. Die Ergebnisse für das erfindungsgemäße Beispiel 1 sowie die nicht erfindungsgemäßen Beispiele 2-4 werden dort im Vergleich zu den nicht erfindungsgemäßen Vergleichsbeispielen V1 und V2 dargestellt. Dabei wird erneut deutlich, dass sämtliche Härtewerte des gezeigten erfindungsgemäßen Beispiels 1 sowie der gezeigten nicht erfindungsgemäßen Beispiele deutlich oberhalb des Wertes für reines Zink angesiedelt sind. Die in Fig. 1 dargestellten Härtewerte liegen dabei zumindest im Bereich des Wertes der bekannten Legierung WE43, wobei die Beispiele 3 und 4 sogar einen sehr viel höheren Härtewert erzielen.

Bezüglich der in Tab. 1 wiedergegebenen Messwerte der Dehngrenze kann festgestellt werden, dass das erfindungsgemäße Ausführungsbeispiel 1 sowie die nicht erfindungsgemäßen Ausführungsbeispiele 2 bis 6 Werte erzielen, welche weit oberhalb des Wertes für Zink (V1) oder PLLA (V3) angesiedelt sind. Ferner zeigt von den Beispielen 1 bis 6 lediglich das erfindungsgemäße Beispiel 1 einen Wert der Dehngrenze, welcher etwas unterhalb des Wertes für WE43 (V2) liegt. Sämtliche übrigen nicht erfindungsgemäßen Beispiele 2-6 zeigen hingegen Werte für die Dehngrenze, welche deutlich oder sogar weit oberhalb des Vergleichswertes für WE43 liegen.

Eine vergleichende grafische Darstellung einer Auswahl der oben erörterten Werte für die Dehngrenze wird überdies in **Figur 2** gezeigt. Hier werden wie in Fig. 1 Messwerte für das erfindungsgemäße Beispiel 1 sowie für die nicht erfindungsgemäßen Beispiele 2 bis 4 mit denjenigen für die Vergleichsbeispiele V1 (Zink) und V2 (WE43) verglichen.

Die mit der erfindungsgemäßen Beispiellegierung 1 sowie den nicht erfindungsgemäßen Beispiellegierungen 2 bis 6 erzielten Werte der Zugfestigkeit sind der fünften Spalte von Tab. 1 im Vergleich mit den korrespondierenden Werten der Vergleichsbeispiele V1 bis V3 entnehmbar. Mit sämtlichen gezeigten Beispiellegierungen werden Werte der Zugfestigkeit erreicht, welche weit oberhalb der Werte für PLLA (V3) und auch Zink (V1) angesiedelt sind. Im Vergleich mit der bekannten Legierung WE43 (V2) liegt lediglich die erfindungsgemäße Legierung gemäß Beispiel 1 knapp unterhalb des Vergleichswerts, sämtliche anderen Legierungen übertreffen die Zugfestigkeit von WE43. Die Beispiele 3 und 4 zeigen dabei Werte der Zugfestigkeit, welche weit oberhalb des Wertes für WE43 (V2) angesiedelt sind.

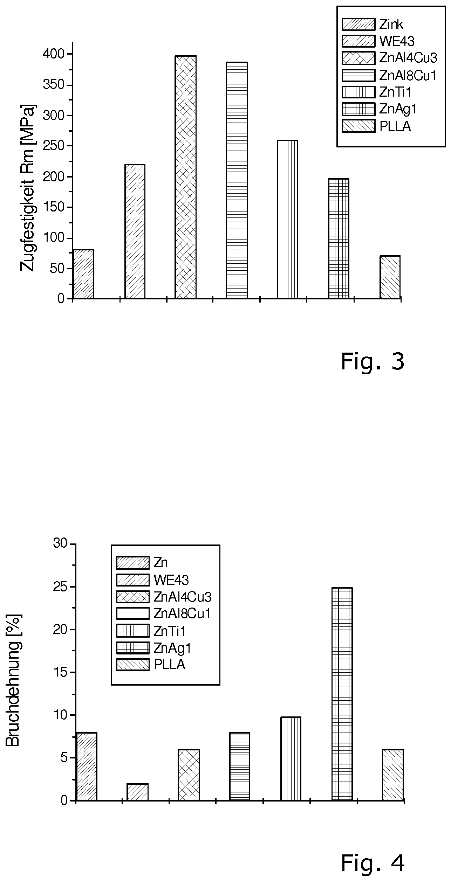

Eine vergleichende grafische Darstellung einer Auswahl der oben erörterten Vergleichswerte für die Zugfestigkeit gemäß Tab. 1 wird überdies in **Figur 3** gezeigt. Hier werden wie in den Fig. 1 und 2 Messwerte für das erfindungsgemäße Beispiel 1 sowie die nicht erfindungsgemäßen Beispiele 2 bis 4 mit denjenigen für die Vergleichsbeispiele V1 (Zink) und V2 (WE43) verglichen. Überdies ist in Fig. 3 der entsprechende Wert für das Vergleichsbeispiel V3 (PLLA) aufgenommen worden.

Die mit der erfindungsgemäßen Beispiellegierung 1 sowie den nicht erfindungsgemäßen Beispiellegierungen 2 bis 6 erzielten Werte der Bruchdehnung im Vergleich mit den korrespondierenden Werten der Vergleichsbeispiele V1 bis V3 sind der rechten Spalte von Tab. 1 entnehmbar. Mit sämtlichen gezeigten Beispiellegierungen werden Werte der Bruchdehnung erreicht, welche deutlich oberhalb des Vergleichswertes für WE 43 (V2) angesiedelt sind. Darüber hinaus zeigen zumindest die Beispiele 1, 2 und 4-6 Werte der Bruchdehnung, welche den Wert für PLLA (V3) deutlich übertreffen. Die mit sämtlichen Beispielen 1-6 erreichten Werte der Bruchdehnung liegen zumindest im Bereich des Verhaltens von Zink (V1), und liegen teils deutlich über diesem Vergleichswert. Die für das erfindungsgemäße Beispiel 1 sowie für die nicht erfindungsgemäßen Beispiele 2 bis 6 belegten hohen Werte der Bruchdehnung wirken sich sehr positiv auf die Eignung der Legierung als Stentmaterial aus, denn beim Dilatieren des Stents gibt es keine Risse und der Stent lässt sich sehr gut in Gefäßkrümmungen führen.

Eine vergleichende grafische Darstellung einer Auswahl der oben erörterten Vergleichswerte für die Bruchdehnung gemäß Tab. 1 wird überdies in **Figur 4** gezeigt. Dabei werden wie in Fig. 3 Messwerte für das erfindungsgemäße Beispiel 1 sowie die nicht erfindungsgemäßen Beispiele 2 bis 4 mit denjenigen für die Vergleichsbeispiele V1 (Zink), V2 (WE43) sowie V3 (PLLA) verglichen.

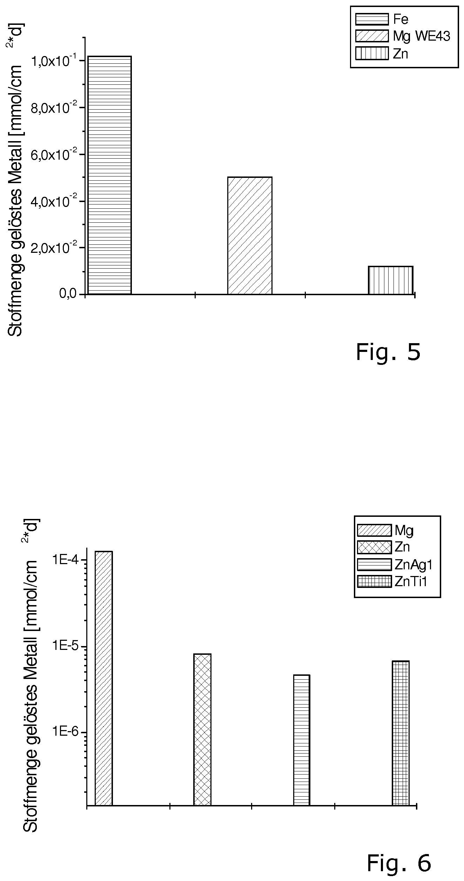

Die weiteren **Figuren 5 und 6** zeigen grafisch dargestellte Ergebnisse von Versuchen zur Bestimmung der Auflösungskinetik sowohl für sortenreine Metalle als auch für vorbekannte Legierungen, eine erfindungsgemäße Legierung und nicht erfindungsgemäße Legierungen. Diese Ergebnisse werden nicht gesondert in tabellarischer Form wiedergegeben.

**Figur 5** zeigt einen Vergleich der Auflösungskinetik der reinen Metalle Eisen und Zink (V1) sowie der bereits erörterten, vorbekannten Magnesiumlegierung WE43 (V2). Dabei wird die Stoffmenge an gelöstem Metall in Millimol bezogen auf einen Quadratzentimeter und einen Tag wiedergegeben.

Zur Bestimmung der in Fig. 5 wiedergegebenen Auflösungskinetik wurden aus den Metallen Eisen, Zink (V1) und der Magnesiumlegierung WE43 (V2) jeweils 1cm² große Flachproben hergestellt. Die Oberfläche dieser Metallproben wurde mit Schleifpapier bis Körnung 1000 poliert und dann in Isopropanol aufbewahrt. In ein verschließbares Plastikgefäß wurden 100ml isotonische Kochsalz-Lösung (0,9g Natriumchlorid / 1l Wasser) abgefüllt, mit einem Rührmagnet versehen und die Proben von dem Deckel hängend in die Lösung eingetaucht. Diese Probenbehälter wurden in einer Temperierkammer auf einen Mehrstellenmagnetrührer gestellt. Die Rührgeschwindigkeit betrug 300 rpm, die Temperatur 37°C. In regelmäßigen Zeitabständen wurde das Medium ausgetauscht und die Konzentration der gelösten Kationen mit der Atomabsorptionsspektroskopie bestimmt.

Die in Fig. 5 gezeigten Ergebnisse belegen eine sehr hohe Auflösungskinetik von Eisen (Fe), welches bekanntlich für einen Einsatz in hier diskutierten Implantaten ungeeignet ist. Die bekannte Legierung WE 43 (V2) zeigt im Vergleich mit Eisen in etwa die halbe Auflösungsgeschwindigkeit, allerdings ist auch die Auflösungskinetik bei WE43 vergleichsweise hoch. Zink (V1) hingegen zeigt eine Auflösungsgeschwindigkeit, welche etwa bei 1/10 des Wertes für Eisen und bei 1/5 des Wertes für WE43 (V2) angesiedelt ist. Dieses Ergebnis belegt eine anfängliche Eignung von Zink für die Herstellung von Implantaten, welche eine vergleichsweise lange andauernde Degradierung in vivo zeigen sollen. Allerdings zeigen die anhand von Tab. 1 diskutierten Messwerte für reines Zink (V1) auf der anderen Seite teils erhebliche Nachteile bei den mechanischen Eigenschaften.

**Figur 6** zeigt einen Vergleich der Auflösungskinetik der reinen Metalle Magnesium und Zink (V1), der erfindungsgemäßen Legierung nach Beispiel 1 (ZnAg1) sowie der nicht erfindungsgemäßen Legierung nach Beispiel 2 (ZnTi1). Dabei wird erneut die Stoffmenge an gelöstem Metall in Millimol bezogen auf einen Quadratzentimeter und einen Tag wiedergegeben.

Zur Bestimmung der in Fig. 6 wiedergegebenen Werte der Auflösungskinetik wurde mit der gleichen Versuchsanordnung wie oben beschrieben vorgegangen. Allerdings sind die Versuche gemäß Fig. 6 in "Simulated Body Fluid" (T. Kokubo, H. Kushitani, S. Sakka, T. Kitsugi and T. Yamamuro, "Solutions able to reproduce in vivo surfacestructure changes in bioactive glass-ceramic A-W", J. Biomed. Mater. Res., 24, 721-734 (1990)) statt in isotonischer Kochsalzlösung durchgeführt worden, da diese Lösung eine vergleichbare Konzentration an Ionen enthält wie Blutplasma.

Für die Herstellung dieser Lösung wurden die folgenden Salze verwendet: NaCl, NaHCO3, KCl, K2HPO4 * 3H2O, CaCl2, Na2SO4, (CH2OH)3CNH2, HCl. Es wurde kein MgCl2 * 6H2O dazugegeben, da Mg ein Bestandteil einiger hergestellter Legierungen ist und damit die Bestimmung der Magnesiumkonzentration verfälscht würde.

Die in Fig. 6 dargestellten Ergebnisse für die Auflösungskinetik belegen erneut die Vorteilhaftigkeit der erfindungsgemäßen Legierungen. Die Auflösungskinetik des hier gezeigten erfindungsgemäßen Beispiels 1 und des hier gezeigten nicht erfindungsgemäßen Beispiels 2 in der simulierten Körperflüssigkeit liegt mindestens eine Größenordnung unter derjenigen für reines Magnesium. Die Legierungen der Beispiele 1 und 2 zeigen in etwa eine vergleichbare Auflösungskinetik wie reines Zink, erweisen sich jedoch hinsichtlich der mechanischen Eigenschaften als dem reinen Zink weit überlegen, wie bereits oben anhand von Tab. 1 sowie den Figuren 1 bis 4 gezeigt werden konnte.

## Patentansprüche

1. Biodegradierbare Legierung, wobei die Legierung aus Zink und Silber besteht, und aufweist (in Massen-%):
| | |
|---|---|
| Zink (Zn): | 90,0 - 99,95% |
| Silber (Ag): | 0,05 - 10,0% |

2. Biodegradierbare Legierung nach Anspruch 1, **gekennzeichnet durch** einen Silbergehalt von 0,1 - 6,0 Massen-% (Ma.-%).

3. Biodegradierbare Legierung nach Anspruch 1 oder 2, **gekennzeichnet durch** einen Silbergehalt von 0,9 - 4,0 Ma.-%.

4. Biodegradierbare Legierung nach einem der Ansprüche 1 bis 3, **gekennzeichnet durch** einen Silbergehalt von 1,0 Ma.-%.

5. Biodegradierbare Legierung nach einem der Ansprüche 1 bis 4, **gekennzeichnet durch** eine oder mehrere der folgenden Eigenschaften (a) bis (g):
(a) Brinellhärte HBW 2,5/62,5 > 50
(b) Dehngrenze (Rp 0,2) > 100 MPa
(c) Zugfestigkeit Rm > 200 MPa
(d) Bruchdehnung > 5%
(e) Auflösezeit im Körper beträgt 1 - 2 Jahre
(f) keine Gefahr einer Emboliebildung im Körper durch Wasserstoffentwicklung
(g) keine Gewebeschädigung im Körper durch Wasserstoffakkumulation

6. Verwendung einer biodegradierbaren Legierung nach einem der Ansprüche 1 bis 5 zur Herstellung oder Beschichtung eines Implantats, insbesondere eines Stents oder eines Knochenimplantats.

7. Implantat, **dadurch gekennzeichnet, dass** das Implantat aus einer biodegradierbaren Legierung nach einem der Ansprüche 1 bis 5 hergestellt oder mit einer solchen Legierung beschichtet ist.

8. Implantat nach Anspruch 7, **dadurch gekennzeichnet, dass** das Implantat ein Stent oder ein Knochenimplantat ist.

9. Verfahren zur Herstellung einer biodegradierbaren Legierung nach einem der Ansprüche 1 bis 5, wobei das Verfahren die Schritte aufweist:
(a) Schmelzen der Legierungsbestandteile unter Schutzgas
(b) Vibrieren der Gussform während des Erstarrens der Schmelze
(c) Erwärmen der Legierung und Halten der Temperatur für definierte Zeiten
(d) Abschrecken der Legierung
(e) Auslagern der Legierung bei definierten Temperaturen
(f) Mechanisches Verformen

10. Verfahren nach Anspruch 9, **dadurch gekennzeichnet, dass** in Schritt (a) das Schutzgas mit einem Überdruck von 1,1-1,3 bar eingesetzt wird.

11. Verfahren nach Anspruch 9 oder 10, **dadurch gekennzeichnet, dass** in Schritt (c) die Legierung in einer Argonatmosphäre bei 100°C-300°C und einer Haltezeit von 0,1 bis 10 Stunden erwärmt wird.

12. Verfahren nach einem der Ansprüche 9 bis 11, **dadurch gekennzeichnet, dass** in Schritt (e) die Legierung in einer Schutzgasatmosphäre bei 50°C bis 150°C ausgelagert wird.

13. Verfahren nach Anspruch 12, **dadurch gekennzeichnet, dass** die Legierung für 0,1 bis 1 Stunde ausgelagert wird.

14. Verfahren nach einem der Ansprüche 9 bis 13, **dadurch gekennzeichnet, dass** in Schritt (f) das mechanische Verformen eine Warmumformung ist und bei 300°C bis 400°C durchgeführt wird.

15. Verfahren nach Anspruch 14, **dadurch gekennzeichnet, dass** die Warmumformung durch Walzen, Strangpressen oder Rundkneten durchgeführt wird.

## Claims

1. A biodegradable alloy, wherein the alloy consists of zinc and silver, and comprising (% by mass):
| | |
|---|---|
| zinc (Zn): | 90.0 - 99.95% |
| silver (Ag): | 0.05 - 10.0% |

2. The biodegradable alloy according to claim 1, **characterized by** a silver content of 0.1 - 6.0 % by mass (ma.-%).

3. The biodegradable alloy according to claim 1 or 2, **characterized by** a silver content of 0.9 - 4.0 ma.-%.

4. The biodegradable alloy according to any of claims 1 to 3, **characterized by** a silver content of 1.0 ma.-%.

5. The biodegradable alloy according to any of claims 1 to 4, **characterized by** one or more of the following properties (a) to (g):
(a) Brinell hardness HBW 2.5/62.5 > 50
(b) yield strength (Rp 0.2) > 100 MPa
(c) tensile strength Rm > 200 MPa
(d) elongation at break > 5%
(e) dissolution time in the body is 1 - 2 years
(f) no risk of embolism formation in the body due to hydrogen evolution
(g) no tissue damage in the body due to hydrogen accumulation.

6. Use of a biodegradable alloy according to any of claims 1 to 5 for the production or coating of an implant, especially a stent or a bone implant.

7. Implant, **characterized in that** the implant is made from a biodegradable alloy according to any of claims 1 to 5 or is coated with such an alloy.

8. The implant according to claim 7, **characterized in that** the implant is a stent or a bone implant.

9. A method for the production of a biodegradable alloy according to any of claims 1 to 5, wherein the method comprises the steps of:
(a) melting the alloy components under protective gas
(b) vibrating the mold during solidification of the melt
(c) heating the alloy and holding the temperature for defined times
(d) quenching the alloy
(e) aging the alloy at specified temperatures
(f) mechanical deformation

10. The method according to claim 9, **characterized in that** in step (a) the protective gas is applied with an overpressure of 1.1 - 1.3 bar.

11. The method according to claim 9 or 10, **characterized in that** in step (c) the alloy is heated in an argon atmosphere at 100°C-300°C and at a holding time of 0.1 to 10 hours.

12. The method according to any of claims 9 to 11, **characterized in that** in step (e) the alloy is aged in a protective gas atmosphere at 50°C to 150°C.

13. The method according to claim 12, **characterized in that** the alloy is aged for 0.1 to 1 hour.

14. The method according to any of claims 9 to 13, **characterized in that** in step (f) mechanical deformation is hot deformation and is carried out at 300°C to 400°C.

15. The method according to claim 14, **characterized in that** hot deformation is carried out by rolling, bar extrusion or rotary swaging.

## Revendications

1. Alliage biodégradable, dans lequel l'alliage se compose de zinc et d'argent, et présente (en % massique) :
| | |
|---|---|
| zinc (Zn) : | 90,0 à 99,95 % |
| argent (Ag) : | 0,05 à 10,0 %. |

2. Alliage biodégradable selon la revendication 1, **caractérisé par** une teneur en argent de 0,1 à 6,0 % massique.

3. Alliage biodégradable selon la revendication 1 ou 2, **caractérisé par** une teneur en argent de 0,9 à 4,0 % massique.

4. Alliage biodégradable selon une des revendications 1 à 3, **caractérisé par** une teneur en argent de 1,0 % massique.

5. Alliage biodégradable selon une des revendications 1 à 4, **caractérisé par** une ou plusieurs des propriétés suivantes (a) à (g) :
(a) dureté Brinell HBW 2,5/62,5 > 50
(b) limite élastique (Rp 0,2) > 100 MPa
(c) résistance à la traction Rm > 200 MPa
(d) allongement à la rupture > 5 %
(e) la durée de décomposition dans le corps se monte à 1 à 2 ans
(f) aucun risque d'une formation d'embolie dans le corps par le développement d'hydrogène
(g) aucun endommagement tissulaire dans le corps par l'accumulation d'hydrogène.

6. Utilisation d'un alliage biodégradable selon une des revendications 1 à 5 pour la fabrication ou l'enrobage d'un implant, notamment d'un stent ou d'un implant osseux.

7. Implant, **caractérisé en ce que** l'implant est fabriqué à partir d'un alliage biodégradable selon une des revendications 1 à 5 ou enrobé d'un tel alliage.

8. Implant selon la revendication 7, **caractérisé en ce que** l'implant est un stent ou un implant osseux.

9. Procédé de fabrication d'un alliage biodégradable selon une des revendications 1 à 5, dans lequel le procédé présente les étapes de :
(a) fonte des constituants de l'alliage sous gaz protecteur
(b) vibration du moule de fonte pendant la solidification de la masse fondue
(c) chauffage de l'alliage et maintien de la température pendant des durées définies
(d) refroidissement brusque de l'alliage
(e) durcissement par précipitation de l'alliage à des températures définies
(f) façonnage mécanique.

10. Procédé selon la revendication 9, **caractérisé en ce que** le gaz protecteur à l'étape (a) est utilisé avec une surpression de 1,1 à 1,3 bar.

11. Procédé selon la revendication 9 ou 10, **caractérisé en ce que** l'alliage à l'étape (c) est chauffé dans une atmosphère d'argon à 100°C à 300°C et pendant une durée de maintien de 0,1 à 10 heures.

12. Procédé selon une des revendications 9 à 11, **caractérisé en ce que** l'alliage à l'étape (e) est durci par précipitation dans une atmosphère de gaz protecteur à 50°C à 150°C.

13. Procédé selon la revendication 12, **caractérisé en ce que** l'alliage est durci par précipitation pendant 0,1 à 1 heure.

14. Procédé selon une des revendications 9 à 13, **caractérisé en ce que** le façonnage mécanique à l'étape (f) est un formage à chaud et est effectué à 300°C à 400°C.

15. Procédé selon la revendication 14, **caractérisé en ce que** le formage à chaud est effectué par laminage, filage ou épointage rotatif.
